# EUROPÄISCHE PATENTSCHRIFT

(11) **EP 2 351 860 B1**
(45) Veröffentlichungstag und Bekanntmachung des Hinweises auf die Patenterteilung: **09.04.2014**
(21) Anmeldenummer: 11003431.1
(22) Anmeldetag: 19.09.2009
(51) Int. Cl.: C21D 8/04, C22C 38/18, C23C 8/26, A61F 2/28, A44C 27/00, G04B 37/22

(54) **Verwendung eines biokompatiblen Werkstoffs aus Edelstahl mit einer martensitischen Randschicht, für Implantate**
Use for implants of a biocompatible material made of stainless steel with a martensitic layer obtained by quenching after nitriding
Utilisation pour implants d'une matière première biocompatible en acier inoxydable dotée d'une couche martensitique obtenue par trempe après nitridation de la surface

(30) Priorität: 08.10.2008 DE 102008050458; 08.10.2008 DE 202008015481 U; 08.12.2008 DE 102008060681
(43) Veröffentlichungstag der Anmeldung: 03.08.2011
(62) Teilanmeldung aus: 09744035.8
(73) Patentinhaber: Dr. Barth, Peter, 89601 Schelklingen (DE)
(72) Erfinder: Dr. Barth, Peter, 89601 Schelklingen (DE)
(74) Vertreter: Pfenning, Meinig & Partner GbR

(56) Entgegenhaltungen:
- EP-A1- 0 652 300
- EP-A1- 1 579 886
- EP-A1- 1 837 414
- EP-A1- 1 956 099
- DE-A1- 4 033 706
- DE-A1-102004 039 926
- ZAUGG R ET AL: "FORTSCHRITTE BEIM STICKSTOFF-EINSATZHAERTEN VON NICHTROSTENDEN STAEHLEN NACH DEM SOLNIT - VERFAHREN", HTM HAERTEREI TECHNISCHE MITTEILUNGEN: ZEITSCHRIFT FUER WERKSTOFFE, WAERMEBEHANDLUNG UND FERTIGUNG, CARL HANSER VERLAG, MUNCHEN, DE, vol. 60, no. 1, 1 January 2005 (2005-01-01), pages 6-11, XP001223297, ISSN: 0341-101X
- IRRETIER, OLAF; RINK, MATTHIAS: "SolNit® - Einsatzhaerten mit Stickstoff von rostbestaendigen Staehlen = SolNit® - nitrogen case hardening of stainless steels", GASWAERME INTERNATIONAL, vol. 55, no. 7/2006, 1 July 2006 (2006-07-01), pages 491-495, XP008157859, ISSN: 0020-9384
- MITSUO NIINOMI ET AL: "Japanese research and development on metallic biomedical, dental, and healthcare materials", JOM, vol. 57, no. 4, 1 April 2005 (2005-04-01), pages 18-24, XP055043444, ISSN: 1047-4838, DOI: 10.1007/s11837-005-0076-3
- DIPPAL MANCHANDA: 'Nickel release regulations, EN 1811-2011- What's new?', [Online] 01 Oktober 2011, Seiten 1 - 6, XP055081018 Gefunden im Internet: <URL:http://www.thelaboratory.co.uk/pdfs/ni ckel release regulations EN1811.pdf> [gefunden am 2013-09-25]

## Beschreibung

Die Erfindung betrifft eine Verwendung eines biokompatiblen Werkstoffs aus Edelstahl, welcher aus einem nichtrostenden, legierten Edelstahl und mit zumindest einer martensitischen Randschicht gebildet ist, für Implante.

Edelstahlschmuck hat in den vergangenen Jahren immer mehr an Bedeutung gewonnen und wird in immer weiterem Umfang angeboten. Uhren aus Edelstahl sind hingegen schon länger bekannt und entsprechend verbreitet auf dem Markt vertreten. Jedoch hat der derzeit verwendete austenitische Stahl, sowohl im Uhren- als auch im Schmuckbereich die äußerst negative Eigenschaft, dass dieser Edelstahl im Gegenteil zu Silber-, Gold-, oder Titanschmuck einen hohen Legierungsbestandteil an Nickel aufweist, der dieses Material für die Herstellung von z.B. Uhren und/oder Schmuck und/oder Implantate sehr negativ erscheinen lässt. Der größte Nachteil hierbei ist der Umstand, dass die meisten Menschen mit allergischen Reaktionen und/oder Empfindungsstörungen auf Nickel reagieren und sie dadurch nickelbelastete Werkstoffe jeglicher Art am oder im Körper vermeiden müssen, um präventiv gegen Allergien vorbeugen zu können.

Ein weiterer Nachteil der neben dem hohen und sehr negativen Nickelgehalt der bisher verwendeten austenitischen Edelstähle für Edelstahlschmuck und/oder Uhren und/oder Implantate ist, dass sich dessen Kratzbeständigkeit, insbesondere dessen Oberflächenhärte (mit ca. 200 HV) zwar durch allgemein und nachstehend beschriebene bekannte Härteverfahren steigern lässt, um ein Verkratzen der hergestellten Produkte z.B. bei Gebrauch bzw. durch Tragen zu vermindern, aber gleichzeitig den extremen Nachteil einer drastischen Erhöhung der Herstellungskosten und zusätzlich noch weitere -nachstehend beschriebene- Nachteile aufweist.

Durch bekannte Verfahren, wie z.B. TiC, TiN, PVD, DLC,.. kann zwar eine Hartstoffschicht auf der Oberfläche des austenitischen Stahls aufgebracht werden, die zwar sehr hart, aber nur sehr dünn (wenige µm) ist. Diese dünnen Schichten haben jedoch den Nachteil, dass sie bei einer mechanischen Beanspruchung, vor allem bei einer mechanischen Punktbeanspruchung einbrechen bzw. abplatzen. Die Begründung liegt darin, dass unter der gehärteten Schicht die Härte schlagartig auf die Ausgangshärte (ca. 200 HV) des verwendeten Stahls abfällt. Dieser Effekt ist in der Literatur als "Eierschaleneffekt" allgemein bekannt.

Des Weiteren können diese austenitischen Stähle zwar mit einer harten und besseren schlagzähen Oberfläche (bis ca. 1800 HV) durch das allg. bekannte Niedertemperatur-Verfahren "Kolsterisieren" nachhaltig gehärtet werden. Hierbei wird bei einer Prozessdauer mit 5 bis 6 Tagen Kohlenstoff bei unter 300 °C eindiffundiert. Der Nachteil dieses Verfahrens ist jedoch die extrem lange Prozessdauer und die damit verbundenen sehr hohen Herstellungskosten. Zudem ist auch hier die relative kleine Schichtdicke mit bis zu maximal 33 µm äußerst negativ, da die Härte innerhalb dieser gradientenbehafteten Schicht relativ schnell bis zur Ausgangshärte des austenitischen Stahls (ca. 200 HV) abfällt. Ein weiterer großer Nachteil dieser gehärteten oder ungehärteten austenitischen Stähle ist deren sehr hoher Nickelgehalt.

Ein Härtungsverfahren eines martensitischen Stahls (AISI 410) ist aus Corrosion Science 48 (2006) 2036-2049 von C. X. Li et al. über ein Plasmanitrierverfahren im Niedertemperaturbereich zwischen 420 - 500 °C bekannt. Nachteilig ist hier wiederum, dass die aufgebrachte Härte mit über 1000 HV fast schlagartig d.h. mit nahezu abruptem Abfall und deutlich erkennbarer Phasengrenze auf die Ausgangshärte abfällt (Eierschaleneffekt) was zur Folge hat, dass eine Stoß- oder Schlagenergie z.B. in Form einer Punktbelastung nur bedingt absorbiert werden kann d.h. eine schlechte Schlagzähigkeit aufweist und damit ein Einbrechen der harten Schicht sehr wahrscheinlich ist. Weiterhin ist aus Davis et al., ASM Handbook, Volume 4, Heating Treating 1991, AMS International US bekannt, auch Stähle des Types AISI 430 und 460 im Niedertemperaturbereich bis maximal 595 °C zu nitrieren.

Bei diesen zitierten Niedertemperaturverfahren (Nitrieren und Plasmanitrieren) für die angegebenen Stähle im Temperaturbereich bis max. 595°C diffundiert zwar Stickstoff in die Stahloberfläche ein, aber es findet keine Phasenumwandlung / Austenitisierung statt. Ein weiterer großer Nachteil diese Stähle nach deren Behandlung ist, dass sie nicht korrosionsbeständig sind. Zusätzlich nachteilig sind die sehr hohen Prozesszeiten zwischen 20 und 48 Stunden und die damit verbundenen deutlich höheren Herstellungskosten.

Bisher galten die ferritischen Chromstähle durch eine Wärmebehandlung aufgrund des zu geringen Kohlenstoffgehalts nach dem allgemeinen Stand der Technik als nicht härtbare und vor allem -im Vergleich zu austenitischen Stählen- auch als nicht korrosionsbeständige Stähle.

Die martensitischen Chromstähle konnten zwar bisher durch bestimmte Härtungsverfahren wie z.B. Einsatzhärtung mit Kohlenstoff gehärtet werden, jedoch nimmt damit der freie Chromanteil und damit dessen Korrosionsbeständigkeit erheblich ab.

Es besteht deshalb ein großes Bedürfnis, einen biokompatiblen Werkstoff aus Edelstahl für alle Produkte die am oder innerhalb des Körpers getragen werden können, noch dahingehend zu verbessern, dass sich der verwendete Chromstahl mit in etwa gleichbleibenden Herstellungskosten neben der geforderten wichtigen Nickelfreiheit und gleichzeitiger ausgezeichneter Kratzbeständigkeit/Härte und Korrosionsbeständigkeit auszeichnet.

Da bei allen Arten von Schmuck, Uhren und Implantaten bzw. allen am oder teilweise innerhalb oder vollständig innerhalb des Körpers getragenen Produkten ein direkter und teilweise auch ein dauerhafter Körperkontakt mit dem verwendeten Werkstoff besteht, ist das Bedürfnis zur Vermeidung von allergischen Reaktionen oder Empfindungsstörungen besonders hoch, ausschließlich einen nickelfreien Werkstoff einzusetzen. Um zusätzlich Verkratzungen und Anrostungen durch Tragen und Gebrauch zu verhindern bzw. zu vermindern, besteht zusätzlich das Bedürfnis einen hoch kratzfesten und korrosionsbeständigen Werkstoff für alle Arten von Schmuck, Uhren und Implantaten einzusetzen. Um die Rentabilität bei den erfindungsgemäßen Gegenständen wie Schmuck, Uhren und Implantaten zu gewährleisten sollten sich die Herstellungskosten nicht erhöhen, sondern vorteilhafterweise in etwa gleich bleiben.

Beim dem vorstehend beschriebenen Stand der Technik können zwar für biokompatible Werkstoffe (wie z.B. Uhren/Uhrenteile, alle Arten von Schmuck und Implantate) auf die derzeit eingesetzten austenitischen Stähle Hartstoffschichten über TiC, TiN, PVD, DLC,.. oder durch Kolsterisieren aufgebracht werden, jedoch sind die damit erzeugten Schichtdicken sehr dünn und brechen bei einer mechanischen Punktbelastung aufgrund des abrupten Abfalls der Härte auf die Ausgangshärte von ca. 200 HV sehr schnell ein oder platzen ab. In diesem Fall sind nicht nur die aufgebrachten Schichten sehr dünn und ohne unterstützende Übergangsschicht nachteilig, sondern die erforderlichen zusätzlichen extrem langen Prozesszeiten und die damit verbundene deutliche Erhöhung der Herstellungskosten. Für biokompatible Werkstoffe, die am oder teilweise innerhalb oder vollständig innerhalb des Körpers getragen werden, wie z.B. alle Arten von Schmuck, Uhren/Uhrenteile und Implantaten, ist mit diesen Verfahren eine wirtschaftliche Produktion nahezu ausgeschlossen.

Bei den beschriebenen Härteverfahren (Nitrieren und Plasmanitrieren) für martensitische Stähle ist zusätzlich der Verlust der Korrosionsbeständigkeit sehr nachteilig und schließt damit eine Anwendung für die erfindungsgemäßen Produkte gänzlich aus.

Ausgehend hiervon ist es die Aufgabe der vorliegenden Erfindung, einen biokompatiblen Werkstoff aus Edelstahl anzugeben, der dem Stand der Technik überlegen ist und der neben in etwa gleich bleibenden Herstellungskosten und zusätzlicher absoluter Nickelfreiheit, eine gleichzeitig so hohe Kratzfestigkeit aufweist, dass ein Verkratzen weitgehend vermieden wird und eine Korrosionsbeständigkeit auch im Dauergebrauch am Körper und/oder teilweise innerhalb des Körpers und/oder vollständig innerhalb des Körpers erreicht wird und allergische Reaktionen und/oder Empfindungsstörungen aufgrund der Nickelfreiheit völlig auszuschließen sind.

Diese Aufgabe wird durch die kennzeichnenden Merkmale des Patentanspruchs 1 gelöst. Die Unteransprüche zeigen vorteilhafte Weiterbildungen auf.

Erfindungsgemäß wird somit vorgeschlagen, dass für den biokompatiblen Werkstoff der Erfindung ein nichtrostender, legierter Edelstahl verwendet wird, wobei die Herstellungskosten nahezu gleich bleiben und sich der Werkstoff vorwiegend am und/oder teilweise innerhalb des Körpers und/oder vollständig innerhalb des Körpers befindet und mit zumindest einer durch eine Wärmebehandlung erzeugten martensitischen Randschicht besteht und der Werkstoff vollständig nickelfrei ist.

Erfindungsgemäß wird unter biokompatiblem Werkstoff aus Edelstahl verstanden, dass es sich hierbei um nichtrostende, legierte Chromstähle, genauer um ferritische und/oder martensitische und/oder ferritisch/martensitische Chromstähle, welche nickelfrei und dadurch allerlei Allergieprobleme, die auf Nickel zurückzuführen sind, auch bei einem lang andauernden oder dauerhaften Körperkontakt außerhalb, als auch innerhalb des Körpers komplett ausgeschlossen werden können.

Unter Nickelfreiheit oder nickelfrei wird hierbei verstanden, dass der eingesetzte Chromstahl keinen Nickel als Legierungsbestandteil enthält.

Bei der Erfindung wird aufgrund der Randschicht ein deutlich höherer Widerstand gegenüber mechanischer Punktbelastung, insgesamt wirtschaftlichere Herstellungskosten, sehr gute Korrosionsbeständigkeit und darüber hinaus, die für die erfindungsgemäßen Produkte überaus wichtige Nickelfreiheit des eingesetzten Werkstoffes gleichzeitig ermöglicht.

Aufgrund des fehlenden Nickelanteils und des damit einhergehenden deutlich billigeren Bezugspreises des erfindungsgemäßen Chromstahls und der zusätzlich besseren Zerspanbarkeit, sind die Herstellungskosten trotz Wärmebehandlung in etwa gleich, als die derzeit für die Gegenstände wie Uhren, Schmuck und Implantaten eingesetzten und unbehandelten austenitischen Stähle.

Durch die Wärmebehandlung wird der Einsatz der erfindungsgemäßen Chromstähle erst möglich, welche sich aufgrund der martensitischen Randschicht in Kombination gleichzeitig durch Nickelfreiheit, Korrosionsbeständigkeit, hoher Kratzfestigkeit bei nahezu gleichbleibenden Herstellungskosten ausweisen. Bei dem Einsatz der erfindungsgemäßen Chromstähle für Gegenstände wie Uhren und Schmuck ist die Nickelfreiheit mindestens genauso wichtig, wie eine hohe Kratzbeständigkeit bei gleichzeitiger Korrosionsbeständigkeit, um Allergiereaktion die auf Nickel zurückzuführen sind durch Gebrauch bzw. durch das Tragen am Körper oder teilweise innerhalb des Körpers komplett ausschließen zu können. Die Kratzbeständigkeit ist bei Implantaten nicht so elementar, jedoch ist auch hier die Nickelfreiheit neben der gleichzeitigen Korrosionsbeständigkeit von äußerster Wichtigkeit.

Unter der erfindungsgemäßen Randschicht, ist diejenige Schicht gemeint, welche orthogonal zur Oberfläche ins Probeninnere eine martensitische Ausbildung des Gefüges und einen nahezu linearen Verlauf der einhergehenden Härtung nach der Wärmebehandlung aufzeigt.
Diese martensitische Randschicht wird durch eine vollständige Phasenumwandlung (sogenannte Austenitisierung) des Gefügezustandes von Ferrit über Austenit zu Martensit durch die Wärmebehandlung erreicht, wobei die Dicke der erzeugten Randschicht abhängig von der Dauer der Wärmebehandlung und dessen Prozessparameter und von der Probendicke ist. Das bedeutet, dass diese martensitische Randschicht bei genügend dünnen Proben auch völlig durchgängig sein kann und sich damit das komplette Gefüge der Probe von Ferrit über Austenit zu Martensit über den kompletten Probenquerschnitt ändert. Der Begriff Randschicht im Sinne der Erfindung umfasst somit auch Ausführungsformen bei der der Werkstoff vollständig aus der Randschicht gebildet ist.
Bei genügend dicken Proben, kann die Schichtdicke des Randbereichs eingestellt und entsprechend an die Bedürfnisse der Bauteile angepasst werden. Typische Randschichtdicken liegen zwischen 80 bis 300 µm.

Selbstverständlich können auch für einen biokompatiblen Werkstoff (z.B. Uhrenboden, Halskette, Ohrring, etc.), welcher Erfahrungsgemäß keine mechanischen Punktbelastungen erfährt, dünnere Randschichten erzeugt werden. Erfindungswesentlich ist jedoch, dass die Randschicht eine Dicke von mindestens 20 µm aufweist, um genügend Schutz bzw. um ohne Verletzung der Randschicht nach der Wärmebehandlung einer vorgenommen Politur oder Mattierung noch ausreichend standhalten zu können.

Die sogenannte Austenitisierung findet bei martensitischen Chromstählen analog zu den ferritischen Chromstählen von Ferrit über Austenit zu Martensit statt, da das Ausgangsgefüge d.h. der unbehandelte martensitische Chromstahl ebenfalls ein rein ferritisches Gefüge aufweist.

Unter einem nahezu linearen Verlauf der einhergehenden Härtung innerhalb der martensitischen Randschicht, ist hierbei gemeint, dass es innerhalb dieser Randschicht einen maximalen Unterschied der Härtewerte um 20 % geben kann, gemessen nach Vickershärteprüfung HV 1.

Die Randschicht kann dabei ein- oder mehrseitig bzw. vorzugsweise den Kern ummantelnd ausgebildet sein, da die Wärmebehandlung vorzugsweise komplett umseitig der Probe erfolgt.

Wenn der Werkstoff eine Randschicht definierter Dicke aufweist, schließt sich daran ein Übergangsbereich an.

Der Übergangsbereich unterscheidet sich in der Gefügeausbildung im Vergleich zum Randbereich zwischen ferritischen und martensitischen Chromstählen.
Beim Übergangsbereich eines ferritischen Chromstahls, ist derjenige Bereich gemeint, welcher sich direkt an die Randschicht ebenfalls orthogonal zur Oberfläche ins Probeninnere anschließt. Bei diesem Übergangsbereich wird keine vollständige Phasenumwandlung aufgrund der Wärmebehandlung erreicht, sondern sie besteht aus einem Mischgefüge des Ausgangsferrits und dem gebildeten Martensit. Das bedeutet, dass in diesem Übergangsbereich keine vollständige Phasenumwandlung durch die Wärmebehandlung aufgrund der tieferen Lage erzielt wird. Die Dicke dieses Übergangsbereichs ist analog zum Randbereich abhängig von der Probendicke und zeigt innerhalb dieses Bereichs eine nahezu lineare Abnahme der einhergehenden Härte und kann über die Dauer der Wärmebehandlung und dessen Prozessparameter eingestellt und entsprechend an die Bedürfnisse der Bauteile angepasst werden.
Beim Übergangsbereich eines martensitischen Chromstahls, ist derjenige Bereich gemeint, welcher sich analog direkt an die Randschicht ebenfalls orthogonal zur Oberfläche ins Probeninnere anschließt. Bei diesem Chromstahl besteht der Übergangsbereich aus einem martensitischen Gefüge mit abnehmender Stickstoffkonzentration in Richtung des Probeninneren. Die Dicke dieses Übergangsbereichs ist analog zum Randbereich des ferritischen Chromstahls abhängig von der Probendicke und zeigt innerhalb dieses Bereichs eine nahezu lineare Abnahme der einhergehenden Härte und kann auch über die Dauer der Wärmebehandlung und dessen Prozessparameter eingestellt und entsprechend an die Bedürfnisse der Bauteile angepasst werden. Typische Übergangsbereiche liegen zwischen 100 bis 600 µm.

Die durch die Wärmebehandlung ausgebildete Randschicht und Übergangszone des biokompatiblen Werkstoffs und der damit verbundenen nicht abrupten, sondern in der Randschicht nahezu linearen und in der Übergangszone kontinuierlich abnehmend Härte, zeigt der erfindungsgemäße Chromstahl eine deutlich höhere Schlagzähigkeit als die bisher bekannten und beschriebenen Härteverfahren.

Die Erfindung schließt dabei auch Ausführungsformen mit ein, bei denen die Randschicht noch einen geringen Anteil, in Abhängigkeit vom eingelagerten C-und N-Gehalt des Stahlwerkstoffs an Restaustenit aufweist.

Unter dem Begriff "Biokompatibler Werkstoff" wird ein Werkstoff verstanden, der am Körper oder mindestens teilweise innerhalb des Körpers angewendet wird.

Bei der verwendeten Bezeichnung "am" Körper, werden hier alle entsprechenden Gegenstände, die dem Fachmann bekannt sind verstanden, die am Körper getragen werden können z.B. Uhren, Uhrenteile wie z.B. Gehäuse, Lünette, Armband, Verschließmechanismen für Uhren und dergleichen, aber auch alle Arten von Schmuck wie z.B. Ringe, Armringe, Armbänder, Ketten und dergleichen.

Bei der verwendeten Bezeichnung "teilweise innerhalb" des Körpers, werden hier alle entsprechenden Gegenstände, die dem Fachmann bekannt sind verstanden, die teilweise innerhalb des Körpers getragen werden können wie z.B. Ohrringe, Ohrenstecker und alle Arten des Piercings und dergleichen.

Bei der verwendeten Bezeichnung "innerhalb" des Körpers, werden hier alle entsprechenden Gegenstände, die dem Fachmann bekannt sind verstanden, die innerhalb des Körper eingebracht werden können z.B. Implantate, insbesondere medizinische Implantate wie z.B. künstliche Gelenke, oder an Knochen angeschraubte stabilisierende Platten, in Knochen integrierte Nägel und dergleichen. Da auch bei allen Arten von Implantaten ein direkter und vor allem ein dauerhafter Körperkontakt mit dem betreffenden Gegenstand besteht und daher ein nickelfreier, korrosionsbeständiger und fester Werkstoff zur Vermeidung von allergischen Reaktionen oder Empfindungsstörungen und zur Herstellung besonders langlebiger Implantate von größtem Vorteil ist.

Übergeordnet können auch die Begriffe "mittelbarer Körperkontakt" und "unmittelbarer Körperkontakt" verwendet werden.

Gemäß der Erfindung besteht der biokompatible Werkstoff somit aus einem nichtrostenden, legierten Chromstahl, der nickelfrei und mit zumindest einer martensitischen Randschicht ausgebildet ist, wobei diese martensitische Randschicht durch eine Änderung des Gefügezustandes von Ferrit über Austenit zu Martensit erreicht wird. Diese martensitische Randschicht zeichnet sich ferner dadurch aus, dass sie orthogonal zur Oberfläche ins Probeninnere, einen nahezu linearen Verlauf der einhergehenden Härte aufzeigt.

Erfindungswesentlich bei dem erfindungsgemäßen biokompatiblen Werkstoff ist zudem, dass der Werkstoff vor der Wärmebehandlung vollständig mechanisch endbearbeitet wird, d.h. sich in einem verkaufsfähigen Zustand befindet.
Die Begründung liegt darin, dass zum Einen eine nach der Wärmebehandlung stattfindende mechanische Bearbeitung (gemeint sind hier alle spanlosen Bearbeitungen, wie z.B. Stanzen und Biegen und alle Bearbeitungen mit Spanbildung wie z.B. Fräsen, Drehen, Schleifen, Bohren) aufgrund der eingestellten Härte nahezu unmöglich würde und zum Anderen eine nachträgliche mechanische Bearbeitung, die erzeugte Randschicht relativ schnell entfernen und/oder durchstoßen könnte und damit der Schutz des erfindungsgemäßen biokompatiblen Werkstoffs gegen Korrosion und Verkratzungen verloren gehen würde.
Das bedeutet, dass z.B. bei bevorzugten polierten und/oder mattierten und/oder aufgerauten Endprodukten eines biokompatiblen Werkstoffes wie z.B. Uhren/Uhrenteile, Schmuck und Implantate, vor der Wärmebehandlung entsprechend endbearbeitet, also poliert bzw. mattiert bzw. aufgeraut sein müssen. Nach der Wärmebehandlung kann -sofern gewünscht- die Oberfläche des Werkstoffes aufgrund eines "leichten Anlaufens" (prozessbedingte Mattierung) noch einmal leicht nachpoliert und/oder mattiert und/oder aufgeraut werden, um die gewünschte Oberfläche vor der Wärmebehandlung wieder zu erhalten. Trotz dieser zusätzlichen Wärmebehandlung und anschließender eventuell vorgenommener Nachbehandlung sind die Herstellungskosten nahezu die Gleichen, wie mit den derzeit eingesetzten austenitischen Stählen für Gegenstände mit mittelbarem bzw. unmittelbaren Körperkontakt, wie Uhren/Uhrenteile, Schmuck und Implantate, da die ferritischen und/oder ferritischen/ martensitischen und/oder martensitischen Chromstähle aufgrund des fehlenden Nickelanteils deutlich kostengünstiger sind.

Gegenstände aus einem solchen biokompatiblen Werkstoff lassen sich vorteilhafterweise aufgrund der Nickelfreiheit am und/oder teilweise innerhalb des Körpers und/oder vollständig innerhalb des Körpers bzw. mittelbar oder unmittelbar am menschlichen Körper anordnen.

Bei dem biokompatiblen Werkstoff ist es dabei bevorzugt, wenn der Härteunterschied zwischen der Oberflächenhärte der Randschicht zur kleinsten Härte des Kerns 130 bis 350 % beträgt. Die Oberflächenhärte der martensitischen Randschicht nach der Wärmebehandlung liegt im Bereich von 500 bis 750 HV 3 und die kleinste Härte des Kerns bei einem ferritischen Edelstahl im Bereich von 160 bis 260 HV 3 und die kleinste Härte des Kerns bei einem martensitischen Edelstahl im Bereich von 400 bis 560 HV 3.
Die nachweisbare Härtesteigerung im Bereich von bis zu 750 HV bringt eine um mindestens 150 fach verbesserte Kratzfestigkeit gemäß dem Kratz- und Verschleißbeständigkeitstests SOP 3-SRC des fem Forschungsinstituts in Schwäbisch Gmünd.

Durch die Verwendung der erfindungsgemäßen Chromstähle erreicht man eine annähernd identische Korrosionsbeständigkeit wie mit austenitischem 1.4301 oder 1.4404 Stählen bei in etwa gleichbleibenden Herstellungskosten und einer um mindestens 15.000% verbesserten Kratzbeständigkeit und der gleichzeitigen wichtigen Nickelfreiheit. Zusätzlich ist die Zerspanbarkeit gegenüber austenitischen Stählen deutlich besser, da sich bei den erfindungsgemäßen Chromstählen ein kürzerer Span ergibt und damit eine automatisierte Zerspannung möglich ist. Damit lassen sich die Herstellungskosten neben der Verwendung des deutlich billigeren erfindungsgemäßen Chromstahls gegenüber dem deutlich teureren austenitischen Stahl zusätzlich verringern.

Sollten die ferritischen oder martensitischen Proben eine Dicke von ca. kleiner 0,7 mm aufweisen, so kann in diesem Fall auch eine komplette Durchhärtung erreicht werden, so dass die Einhärtungstiefe der Randschicht komplett durchgängig ist und damit diese Proben ausgehend von der Oberfläche bis hin zum Kern und damit über den gesamten Probequerschnitt einen nahezu linearen Härteverlauf d.h. in diesem Fall einen durchgängigen martensitischen Bereich aufzeigen, da die Wärmebehandlung beidseitig bzw. allseitig erfolgt.

Auch ist bei ferritischen Proben mit einer Dicke zwischen größer ca. 0,7 mm bis ca. 3 mm denkbar, da die Wärmebehandlung beidseitig bzw. allseitig erfolgt, dass es keinen reinen ferritischen Kernbereich mit entsprechender Ausgangshärte des unbehandelten Werkstoffes mehr gibt, sondern in diesem Fall die Probe durch das Einlagern von Stickstoff und dem darauffolgenden Abschrecken in einer Zwischenhärte (Mischhärte) zwischen Martensithärte und Kernhärte, d.h. bei einer höheren Härte endet. Die Eindringtiefe des äußeren martensitischen Bereichs (Randschicht) bleibt in etwa gleich, aber der ferritisch-martensitische Bereich (Übergangsbereich) verkürzt sich aufgrund der höheren Mischhärte und des linearen Abfalls des Übergangsbereichs. Analog gilt dies für martensitische Proben mit dem Unterschied, dass hier der Übergangsbereich martensitisch mit abnehmender Stickstoffkonzentration ausgebildet ist und die Härte entsprechend in einer Zwischenhärte größer der Ausgangshärte endet.

Bei dem biokompatiblen Werkstoff ist es bevorzugt, wenn die Oberfläche der martensitischen Randschicht aufgeraut und/oder mattiert und/oder poliert und/oder unbehandelt ist. Die Oberflächenrauhigkeit kann dabei im Bereich von 0,01 µm bis 4,0 µm liegen.

Bleibt die Oberfläche des biokompatiblen Werkstoffs nach der Wärmebehandlung unbehandelt, d.h. sie wird nicht poliert, mattiert oder aufgeraut, erhöht sich die Kratzfestigkeit noch weiter, da sich höhere Härtespitzen an der Oberfläche nach der Wärmebehandlung einstellen und nicht abgetragen werden. Durch den Entfall einer anschließenden Oberflächenbehandlung könnten die variablen Kosten noch weiter gesenkt und eine neuartige Oberflächenausführung (Bezeichnung: z.B. "satiniert" oder "new satin") erreicht werden.

Aus stofflicher Sicht können bei dem erfindungsgemäßen biokompatiblen Werkstoff im Prinzip alle nichtrostenden, legierten Edelstähle, die nickelfrei und nicht austenitisch sind, verwendet werden. Beispiele hierfür sind alle Stahlgruppen mit den Werkstoffnummern 1.40xx, 1.41xx, 1.45xx, 1.46xx und 1.47xx welchen kein Nickel zulegiert wurde.
Die Buchstaben x geben hier jeweils eine Zahl zwischen 0 und einschließlich 9 -gemäß dem Stahlschlüssel, erweiterte Auflage 2007- an.
Beispiele: 1.4000, 1.4001, 1.4002, 1.4005, 1.4007, 1.4009, 1.4010, 1.4015, 1.4016, 1.4021, 1.4024, 1.4028, 1.4029, 1.4031, 1.4034, 1.4035, 1.4036, 1.4037, 1.4085, 1.4086, 1.4104, 1.4105, 1.4106, 1.4109, 1.4110, 1.4111, 1.4112, 1.4113, 1.4116, 1.4117, 1.4119, 1.4125, 1.4126, 1.4133, 1.4136, 1.4138, 1.4153, 1.4509, 1.4510, 1.4511, 1.4513, 1.4520, 1.4521, 1.4523, 1.4525, 1.4526, 1.4528, 1.4535, 1.4590, 1.4592, 1.4595, 1.4601, 1.4602, 1.4603, 1.4604, 1.4605, 1.4724, 1.4725, 1.4735, 1.4736, 1.4742, 1.4746 1.4748, 1.4749, 1.4760, 1.4761, 1.4762, 1.4763, 1.4765, 1.4767, 1.4768 und 1.4783.
Selbstverständlich sollen auch alle zukünftigen derzeit noch nicht in den Stahlschlüssel aufgenommen Edelstähle hiermit erfasst sein.
Bevorzugt sind die Stähle 1.4016 oder 1.4021.

Durch eine martensitische Randschicht resultiert eine deutliche Härtesteigerung, womit dann überlegene Eigenschaften in Bezug auf Kratz-und Korrosionsbeständigkeit, die neben der äußerst wichtigen Nickelfreiheit bei in etwa gleich bleibenden Herstellungskosten erreicht werden.

Die Ausbildung der martensitischen Randschicht kann bei dem biokompatiblen Werkstoff durch eine Wärmebehandlung, bevorzugt durch ein sog. "Aufsticken" erfolgen. Der Chromstahl muss vorzugsweise einen Chromanteil von mindestens 12% aufweisen. Das Aufsticken von Stahlwerkstoffen ist an und für sich im Stand der Technik bekannt und wird z.B. in der EP 0 652 300 A1 oder auch in der DE 40 33 706 beschrieben.

Beim Randaufsticken wird dabei so vorgegangen, dass der Stahlwerkstoff bei einer Temperatur zwischen 1000 °C und 1200 °C in einer stickstoffhaltigen Gasatmosphäre zwischen 3 - 6 Stunden und nachfolgender Abkühlung behandelt wird.

Es hat sich nun überraschenderweise gezeigt, dass ein derartiges Verfahren bei der Anwendung auf einen vollständig mechanisch endbearbeiteten biokompatiblen Werkstoff, d.h. auf einen nickelfreien Chromstahl zu überlegenen Eigenschaften führt.

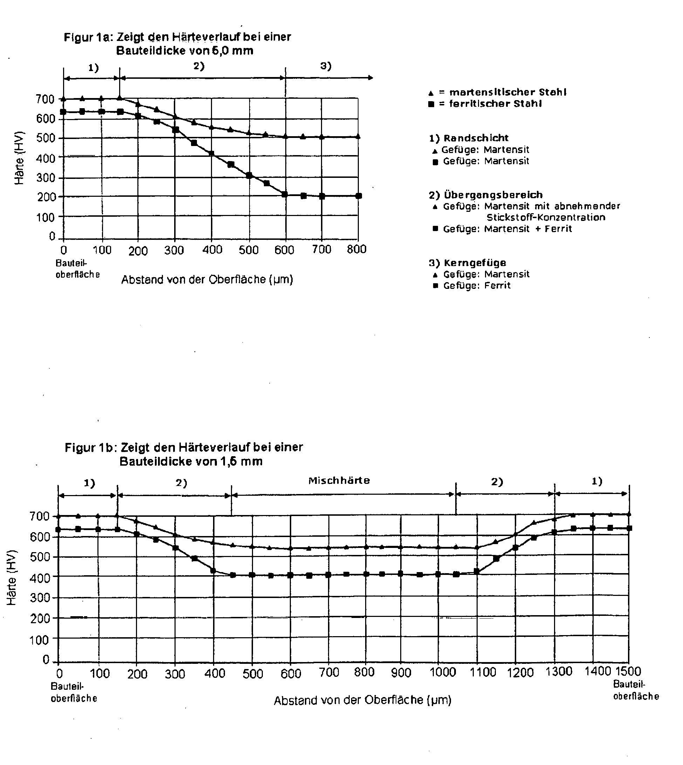

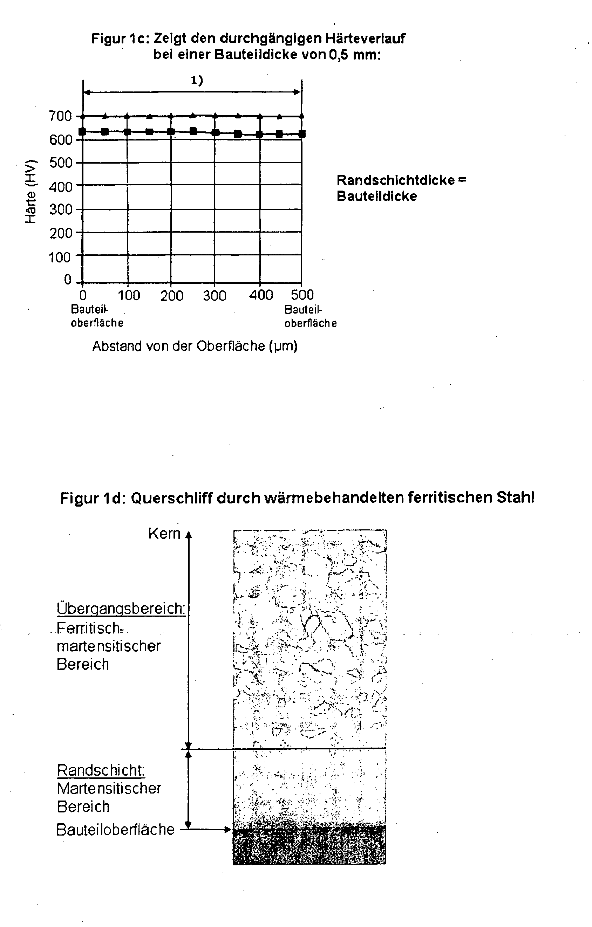

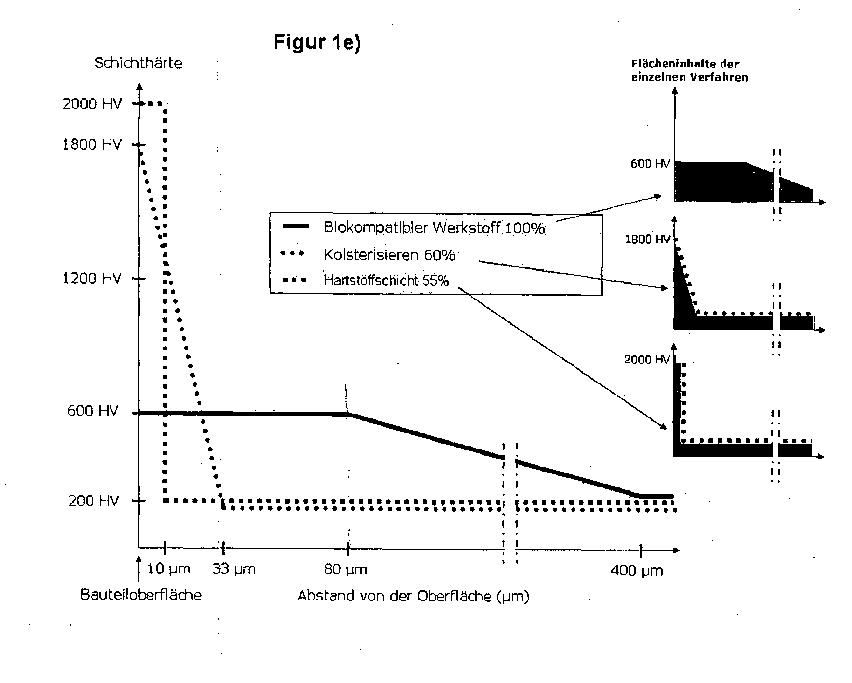

Die Erfindung wird nachfolgend anhand der Figuren 1a, 1b, 1c, 1d und 1e näher erläutert, ohne den Gegenstand der Erfindung hierauf zu beschränken. Die Schaubilder sind idealisiert dargestellt und können selbstverständlich in ihrem Verlauf von der dargestellten Form abweichen.

Die Figur 1 a zeigt den Härteverlauf nach einer Wärmebehandlung am Beispiel eines ferritischen Chromstahls und eines martensitischen Chromstahls in Form einer graphischen Darstellung.

Bei martensitischen Chromstählen ist die Randschicht äquivalent zu der bei ferritischen Chromstählen, d.h. sie besteht aus einem martensitischen Gefüge. Die Übergangszone hingegen unterscheidet sich, da sie in diesem Fall kein ferritisches-martensitisches Gefüge sondern sich durch ein martensitisches Gefüge mit zum Probeninneren hin abnehmender Stickstoffkonzentration auszeichnet.

Im Beispielsfall nach der Figur 1a wurde eine ferritische (1.4016) und eine martensitische Probe (1.4021) mit einer Dicke von 5 mm bei Temperaturen von über 1050 °C mit Stickstoff aufgestickt und abgeschreckt bzw. tiefgekühlt und angelassen.

Der Werkstoff 1.4016, X6 Cr 17 ist ein ferritischer Chromstahl mit kleiner gleich 0.08 % Kohlenstoff. Durch das Einlagern von Stickstoff kommt es zu einer Umwandlung in Austenit, wobei beim darauffolgenden Abschrecken Martensit entsteht, der wie aus Figur 1d zu sehen ist zum Kern hin entsprechend der Einlagerungen abnimmt. Offensichtlich wird durch die hohe Behandlungstemperatur mit variablen Prozessdrücken und der Stickstoffaufnahme eine nahezu vollständige Phasenumwandlung, d.h. eine sogenannte Austenitisierung erreicht. Dabei wandelt sich die kubisch raumzentrierte Tieftemperaturphase α (Ferrit) vollständig in die kubisch flächenzentrierte Hochtemperaturphase γ (Austenit) um. Die nachfolgende Abschreckung/Unterkühlung erfolgt so schnell, dass Diffusionsprozesse ausbleiben und dass das Gefüge in Martensit umklappt. Dieses stark verspannte Gitter führt zu der schon geschilderten Härtesteigerung des Werkstoffes. Im Ergebnis führt dies dazu, dass eine komplette Gefügeänderung zumindest im oberflächennahen Bereich eintritt. Dies geht auch aus den Beispielen, insbesondere aus der Figur 1 a hervor. Wie dieses Beispiel anschaulich zeigt, ist der Härteverlauf des erfindungsgemäßen Stahlwerkstoffes bei hinreichend dicken Proben mit größer ca. 3 mm derart ausgebildet, dass hierbei ausgehend von der Oberfläche in Richtung des inneren Ferritkernes im martensitischen Randbereich eine nahezu lineare und in der ferritisch-martensitischen Übergangszone eine kontinuierliche Veränderung des Gefügezustandes und damit auch der damit einhergehenden Härteänderung erfolgt.
Wie aus Figur 1 a hervorgeht, weist die ferritische Probe aus 1.4016 eine Oberflächenhärte von ca. 650 HV 3 auf. Die Einhärtungstiefe beträgt im Beispielsfall 150 µm. Die Berechnung der Einhärtungstiefe wird erfindungsgemäß so durchgeführt, dass von der geringsten Härte des Kerns ebenfalls gemessen in HV 3 + 30 % ausgegangen wird. Im Beispielsfall ist somit der Ausgangswert 200 HV 3. Die Randschicht einer solchen Probe mit einer Dicke von größer ca. 3 mm im Beispielfall 5 mm zeigt ausgehend von der Oberfläche in Richtung der Probenmitte innerhalb der Randschicht (martensitischer Bereich =1)) einen nahezu linearen Härteverlauf und in der daran anschließenden Übergangszone (martensitisch-ferritischer Bereich =2)) eine kontinuierliche Abnahme der einhergehenden Härtung bis auf die Kernhärte (ferritischer Bereich =3)) im Beispielfall 200 HV 3.
Die Figur 1a zeigt weiterhin den Härteverlauf eines martensitischen Chromstahls. Dieser Werkstoff 1.4021, X20 Cr 13 ist ein martensitischer Chromstahl mit 0,16 bis 0,25 % Kohlenstoff. Im Vergleich zum ferritischen Chromstahl weist dieser Stahl nach einhergegangener Wärmebehandlung aufgrund des höheren Kohlenstoffanteils eine etwas größere lineare Härte mit bis zu 750 HV (im Beispielfall ca. 700 HV 3) innerhalb der Randschicht = 1) und eine analoge kontinuierliche Abnahme der Härte innerhalb der Übergangszone = 2) bis auf dessen Kernhärte (nach der Wärmebehandlung) von ca. 500 HV 3 auf.

In beiden Fällen wird damit ein großer Vorteil gegenüber den im Stand der Technik beschriebenen Verfahren deutlich, welche eine abrupte Veränderung des Härteverlaufes und des Gefügezustandes d.h. eine Phasengrenze aufzeigen.

Aus Figur 1b geht hervor, dass es bei einer ferritischen Probe aus 1.4016 mit einer Dicke zwischen ca. 0,7 mm und kleiner ca. 3 mm im Beispielfall 1,5 mm aufgrund der beidseitigen bzw. allseitigen Wärmebehandlung möglich ist, dass es keinen ferritischen Kernbereich mehr gibt, sondern die Härte im Probeninneren durch das Einlagern von Stickstoff und dem darauffolgenden Abschrecken in einer Zwischenhärte (= Mischhärte) zwischen Martensithärte und Ausgangshärte endet. D.h. die Kernhärte nimmt in diesem Fall nicht bis auf die Ausgangshärte des unbehandelten ferritischen Werkstoffes ab, sondern endet entsprechend bei einer höheren Härte (im Beispielsfall 400 HV 3) und steigt anschließend im Querschnitt wieder kontinuierlich auf die Martensithärte der Randschicht =1) im Beispielfall 650 HV 3 an. Der martensitische Bereich = Randbereich = 1) bleibt im Beispielfall mit einer Eindringtiefe von ca. 150 µm gleich, der Übergangsbereich = 2) verkürzt sich jedoch aufgrund der höheren Zwischenhärte (= Mischhärte) des Probeninneren und des linearen Abfalls des Übergangsbereichs von ca. 450 µm auf ca. 300 µm. Analoges gilt für martensitische Proben, nur das sich hier im Beispielfall die Zwischenhärte (= Mischhärte) im Probeninneren auf ca. 550 HV einstellt und anschließend analog im Querschnitt wieder kontinuierlich auf die Martensithärte der Randschicht = 1) im Beispielfall 700 HV 3 ansteigt. Der martensitische Bereich = Randbereich = 1) bleibt im Beispielfall mit einer Eindringtiefe von ca. 150 µm ebenfalls gleich, der Übergangsbereich = 2) verkürzt sich analog aufgrund der höheren Zwischenhärte (= Mischhärte) des Probeninneren und des linearen Abfalls des Übergangsbereichs.

Wie aus Figur 1c hervorgeht, ist es bei genügend dünnen ferritischen Proben aus 1.4016 mit einer Dicke von ca. kleiner 0,7 mm im Beispielfall 0,5 mm selbstverständlich möglich, dass eine komplette Durchhärtung erreicht wird, d.h. die Einhärtungstiefe der Randschicht ist komplett durchgängig und martensitisch = 1), so dass diese Proben ausgehend von der Oberfläche bis hin zum Kern und damit über den gesamten Probenquerschnitt einen durchgehenden nahezu linearen, d.h. einen konstanten Härteverlauf zeigen, im Beispielfall 650 HV 3. Analoges gilt für martensitische Proben dieser Größenordnung, d.h. auch hier wird eine komplette Durchhärtung mit konstantem Härteverlauf im Beispielfall 700 HV 3 erreicht.

Grundsätzlich ist zur Fig. 1a zu sagen, dass natürlich abhängig von der Bauteilgröße und der Abkühlgeschwindigkeit noch andere Phasen wie z.B. Perlit und Bainit im Kerngefüge =3) möglich sein können.

Figur 1d zeigt in einem Querschliff einer wärmebehandelten ferritischen Probe aus 1.4016 in der Vergrößerung 50:1 sehr anschaulich die Gefügeausbildung, aus der die martensitische Randschicht zu erkennen ist. Gleichfalls ist die Übergangszone aus einer Mischung aus Ferrit- und Martensitkörnern sehr gut zu erkennen. Die behandelte Oberfläche weist dabei einen mittleren Korndurchmesser von 28 bis 40 µm gemessen nach dem Linienschnittverfahren auf. Der Korndurchmesser des behandelten Teils liegt im Kern bei 15 bis 20 µm und der des unbehandelten Ausgangsmaterials bei 10 bis 14 µm.
Die Querschliffabbildung einer wärmebehandelten martensitischen Probe wurde nicht vorgenommen, da aufgrund des durchgängigen martensitischen Gefüges weder die Randschicht und Übergangszone noch der Kern zu unterscheiden wären.
Figur 1e zeigt zum Einen im direkten Vergleich der derzeitigen bekannten -und schon ausreichend beschriebenen Verfahren (Hartstoffschichten, Kolsterisieren) und dem hier dargestellten neuen Verfahren bzgl. eines biokompatiblen Werkstoffs und die damit realisierbaren durchschnittlichen Schichtdicken mit den entsprechenden Härteverläufen. Und zum Anderen werden die jeweiligen integrierten Flächeninhalte der beschriebenen Verfahren, die ein Maß für den Widerstand gegen Punktbelastung (Schlagzähigkeit) widerspiegeln, dargestellt. Hierbei wurde der Flächeninhalt, der bei dem biokompatiblen Werkstoff erreicht wird mit 100% festgelegt und die anderen Verfahren entsprechend ihren Flächeninhalten dagegen gerechnet. Wie daraus deutlich zu erkennen ist, kann der hier beschriebene biokompatible Werkstoff einer tendenziell um 40% größeren mechanischen Punktbelastung standhalten, als die anderen beiden Verfahren, die einem nahezu abrupten Abfall d.h. eine Phasengrenze der einhergegangenen Härtung aufzeigen.

Überraschenderweise wurde nun festgestellt, dass der biokompatible Werkstoff mit der vorstehend beschriebenen Oberflächenausbildung durch eine oberflächennahe Phasenumwandlung / Austenitisierung überlegene Korrosionsbeständigkeiten und Kratzbeständigkeiten bei gleichzeitiger gewünschter absoluter Nickelfreiheit und in etwa gleich bleibenden Herstellungskosten aufweist.

Im Ergebnis ist somit festzustellen, dass der biokompatible Werkstoff aus einem nichtrostenden, legierten Chromstahl besteht, der absolut Nickelfrei und bei dem abhängig von der Probendicke jedoch zumindest die Randschicht martensitisch ausgebildet ist, wobei die martensitische Randschicht orthogonal zur Oberfläche ins Probeninnere einen nahezu linearen Verlauf der einhergehenden Härtung aufzeigt und durch eine Änderung des Gefügezustandes von Ferrit über Austenit zu Martensit erreicht wird und der entsprechende Werkstoff vorzugsweise am und/oder teilweise innerhalb des Körpers und/oder vollständig innerhalb des Körpers bzw. mittelbar oder unmittelbar mit dem menschlichen Körper in Kontakt steht. Darüber hinaus zeichnet sich der erfindungsgemäße biokompatible Werkstoff durch nahezu gleich bleibende Herstellungskosten aus. Der erfindungsgemäße biokompatible Werkstoff der vorzugsweise am und/oder teilweise innerhalb des Körpers und/oder vollständig innerhalb des Körpers bzw. mittelbar oder unmittelbar mit dem menschlichen Körper in Kontakt steht zeichnet sich also vorwiegend dadurch aus, dass er in Kombination gleichzeitig gleichbleibende Herstellungskosten, Nickelfreiheit, hohe Kratzbeständigkeit und Korrosionsbeständigkeit aufweist.

## Patentansprüche

1. Verwendung eines biokompatiblen Werkstoffes aus einem nichtrostenden, legierten Edelstahl und zumindest einer durch eine Wärmebehandlung unter Aufstickung und nachfolgender Abkühlung gebildeten martensitischen Randschicht für Implantate, wobei orthogonal zur Oberfläche ins Probeninnere, die von der Probendicke abhängige martensitische Randschicht einen linearen Verlauf der einhergehenden Härtung aufzeigt und der Edelstahl nickelfrei ist.

2. Verwendung nach Anspruch 1, für künstliche Gelenke oder an Knochen angeschraubte stabilisierende Platten oder in Knochen integrierte Nägel.

3. Verwendung nach Anspruch 1 oder 2, **dadurch gekennzeichnet, dass** die martensitischen Randschicht durch eine vollständige Phasenumwandlung des Gefügezustandes von Ferrit über Austenit zu Martensit erreicht wird.

4. Verwendung nach Anspruch 1, **dadurch gekennzeichnet, dass** der Werkstoff vor der Wärmebehandlung vollständig mechanisch endbearbeitet wird, d.h. sich in einem verkaufsfähigen Zustand befindet.

5. Verwendung nach Anspruch 1, **dadurch gekennzeichnet, dass** die martensitische Randschicht eine Dicke von mindestens 20 µm aufweist.

6. Verwendung nach mindestens einem der Ansprüche 1 bis 5, **dadurch gekennzeichnet, dass** der Stahlwerkstoff ausgewählt ist aus den Stahlgruppen mit den Werkstoffnummern 1.40xx, 1.41xx, 1.45xx, 1.46xx und 1.47xx.

7. Verwendung nach mindestens einem der Ansprüche 1 bis 6 mit einer zusätzlichen Übergangszone, **dadurch gekennzeichnet, dass** die Übergangszone eine kontinuierliche Abnahme der einhergehenden Härtung aufzeigt.

8. Verwendung nach mindestens einem der Ansprüche 1 bis 7, **dadurch gekennzeichnet, dass** die Oberflächenhärte der Randschicht im Bereich von 500 bis 750 HV 3 liegt.

## Claims

1. Use of a biocompatible material made of a rust-resistant, alloyed stainless steel and at least one martensitic surface layer which is formed by a heat treatment with nitrogen case hardening and subsequent cooling for implants, **characterised in that**, orthogonally to the surface into the sample interior, the martensitic surface layer displays a virtually linear course of the accompanying hardening and the stainless steel is nickel-free.

2. Use according to claim 1 for artificial joints or stabilised boards screwed at bones or integrated nails in bones.

3. Use according claim 1 or 2, **characterized in that** the martensitic surface layer is produced by a complete phase change of the structural state from ferrite via austenite to martensite.

4. Use according to claim 1, **characterized in that** the material is completely mechanically finished before the heat treatment, i.e. is in a ready-for-sale state.

5. Use according to claim 1, **characterized in that** the martensitic surface layer has a thickness of at least 20 µm.

6. Use according to one of the claims 1 to 5, **characterized in that** the steel material is selected from the steel groups with the material numbers 1.40xx, 1.41xx, 1.45xx, 1.46xx and 1.47xx.

7. Use according to one of the claims 1 to 6, having an additional transition zone, **characterized in that** the transition zone displays a continuous decrease in the accompanying hardening.

8. Use according to one of the claims 1 to 7, **characterized in that** the surface hardness of the surface layer is in the range of 500 to 750 HV3.

## Revendications

1. Utilisation pour des implants d'un matériau biocompatible constitué d'un acier noble allié inoxydable et d'au moins une couche superficielle martensitique formée par un traitement thermique avec nitruration suivie d'un refroidissement, la couche superficielle martensitique dépendante de l'épaisseur de l'échantillon présentant, orthogonalement par rapport à la surface et dans la direction de l'intérieur de l'échantillon, une variation linéaire de la dureté correspondante et l'acier spécial étant exempt de nickel.

2. Utilisation selon la revendication 1 pour des articulations artificielles ou des plaques de stabilisation vissées sur des os, ou des clous intégrés à des os.

3. Utilisation selon la revendication 1 ou 2, **caractérisée en ce que** la couche superficielle martensitique est obtenue par une transformation de phase complète de la structure, d'un état ferritique à un état martensitique en passant par un état austénitique.

4. Utilisation selon la revendication 1, **caractérisée en ce que** le matériau est, avant le traitement thermique, soumis à un usinage final entièrement mécanique, c'est-à-dire se trouve dans un état prêt à la commercialisation.

5. Utilisation selon la revendication 1, **caractérisée en ce que** la couche superficielle martensitique présente une épaisseur d'au moins 20 µm.

6. Utilisation selon au moins l'une des revendications 1 à 5, **caractérisée en ce que** le matériau de type acier est choisi parmi les groupes d'aciers portant les numéros de matériaux 1.40xx, 1.41xx, 1.45xx, 1.46xx et 1.47xx.

7. Utilisation selon au moins l'une des revendications 1 à 6, comportant une zone de transition supplémentaire, **caractérisée en ce que** la zone de transition présente une diminution continue de la dureté correspondante.

8. Utilisation selon au moins l'une des revendications 1 à 7, **caractérisée en ce que** la dureté surfacique de la couche superficielle est comprise dans la plage de 500 à 750 HV 3.
